# EUROPEAN PATENT APPLICATION

(11) **EP 3 995 828 A1**
(43) Date of publication of application: **11.05.2022**
(21) Application number: 20205766.7
(22) Date of filing: 04.11.2020
(51) Int. Cl.: G01N 33/543, B01L 3/00, C12M 1/00, C08J 7/04, C08J 7/056, C12N 11/08, C12M 1/12

(54) **NOVEL FUNCTIONALIZED HYDROGEL COATINGS OF ASSAY PLATES AND USES THEREOF**

(71) Applicant: MicroCoat Biotechnologie GmbH, 82347 Bernried (DE)
(72) Inventor: STRAUB, Alexander Johannes, 79106 Freiburg (DE); RÜHE, Jürgen, 79356 Eichstetten (DE); BRANDSTETTER, Thomas, 79110 Freiburg (DE); SCHERAG, Frank Daniel, 79114 Freiburg (DE); KIND, Martin, 87437 Kempten (DE); STEINER, Mark-Steven, 82362 Weilheim (DE)
(74) Representative: Grünecker Patent- und Rechtsanwälte PartG mbB

(57) **Abstract**

The present invention relates to the field of microplate assay-based analytical and diagnostic applications, in particular immunoaffinity assays, ELISA or ELISpot assays. More specifically, the present invention relates to functionalized hydrogel coatings of assay plates. The present invention provides a novel method of manufacturing a surface-bonded continuous layer of a copolymer that can be applied to assay plates used in analytical or diagnostic applications. The copolymer is characterized in that it comprises at least three different monomer units and includes at least one photo-reactive side group for a C, H-insertion cross-linking ["CHic"] reaction; and at least one click binding-reactive side group ["Click"] for (bio-) functionalizing of the copolymer layer. Furthermore, the present invention provides containers having such a novel surface-bonded continuous layer of a copolymer, as well as uses thereof for the selective attachment and/or capturing of biochemical reagents to the copolymer layer.

## Description

### FIELD OF THE INVENTION

The present invention relates to the field of microplate assay-based analytical and diagnostic applications, in particular immunoaffinity assays, ELISA or ELISpot assays. More specifically, the present invention relates to functionalized hydrogel coatings of assay plates. The present invention provides a novel method of manufacturing a surface-bonded continuous layer of a copolymer that can be applied to assay plates used in analytical or diagnostic applications. The copolymer is characterized in that it comprises at least three different monomer units and includes at least one photo-reactive side group for a C, H-insertion cross-linking ["CHic"] reaction; and at least one click binding-reactive side group ["Click"] for (bio-) functionalizing of the copolymer layer. Furthermore, the present invention provides containers having such a novel surface-bonded continuous layer of a copolymer, as well as uses thereof for the selective attachment and/or capturing of biochemical reagents to the copolymer layer.

### BACKGROUND OF THE INVENTION

Testing samples for the presence of analytes of interest can be important in a variety of applications, including disease diagnostics, food and water safety and environmental surveillance. A variety of analytical methods can be performed on samples to determine if a sample contains a particular analyte.

One area of application of such analytical methods is in pharmaceutical companies nowadays applying high throughput drug discovery systems which enable automated, highly miniaturized screening of hundreds of thousands of samples to discover pharmacologically active compounds interacting with therapeutic targets on the molecular level. There is a strong need for a reliable early-on evaluation of the pharmacological potential of these compounds in order to determine the most promising candidates for the costly drug discovery. As a lot of compounds are available in only small amounts, it is of particular importance that the techniques utilized provide highly specific and sensitive detection of the analyte of interest. There is also a continued need for sensitive diagnostic and prognostic methods of detecting analytes. For example, an important parameter in tuberculosis diagnostics (interferon gamma) is detected and quantified millions of times in diagnostic laboratories worldwide. There is also high demand for diagnostic applications in cancer samples that may or may not contain cancer cells and to assess the level of cancer in patients before, during and/or after treatment. Similarly, autoimmune diseases is another area in which there exists a continued need for diagnostic, prognostic, and therapeutic analytical methods.

Such analytical methods are carried out on assay substrates. An assay substrate is a surface upon which various chemical and/or biological analyses can be performed. Examples of assay substrates include microarray plates, glass plates, and microtiter plates. A microtiter plate is a flat plate that has multiple containers or "wells" formed in its surface. Each well can be used as a small test tube into which various materials can be placed to perform biochemical analyses. One illustrative use of microtiter plates includes enzyme-linked immunosorbent assay (ELISA).

In recent years, the target of cell analysis has become subdivided from cell colony level into one cell level. Attempts are being made to capture cells one by one, identify, sort, collect, and culture the cells, and perform analytical techniques. As the method for identifying and capturing the cells, a method is adopted in which secretions from the cells are separated, target secretions are detected, and then the cell secreting the target secretions is screened out. For the single-cell analysis described above, a technique of comprehensively capturing and analyzing cells is required. The ELISpot (Enzyme-linked-immuno-Spot) test is a sensitive method to measure cell activation at the single cell level. This cell activation releases cytokines, which are then made visible as colored dots (spots) in the ELISpot using monoclonal antibodies. A well-known problem with analytical techniques such as ELISpot technology is the creation of clearly defined spots after the detection reaction has been completed. Spot generation is influenced, among other things, by high non-specific background signals, a "washing out" of the edges and the mixing of the individual spots into clusters. Here, however, cells that are close together can also cause interference of the spots that form.

Therefore, there is a need in the art for high immobilized concentrations of the capture antibodies leading to a rapid local binding of the antigens secreted by the cells in the immediate vicinity of the cell body, which minimizes the problems with sensitivity such as the washing out of the spots in the ELISpot technology and reducing so-called "specific background signals" on the detection surface. Furthermore, there is a need to combine such sensitive analytical techniques with their ability to specifically capture, separate and identify the secretions by individual cells.

### SUMMARY OF THE INVENTION

The present invention relates to functionalized hydrogel coatings of assay plates. The present invention provides a novel method of manufacturing a surface-bonded continuous layer of a copolymer that can be applied to assay plates used in analytical or diagnostic applications. The present invention further provides a container comprising such a layer and uses thereof. The present invention involves the techniques of hydrogels, click chemistry, and photomasks for photolithographic structuring using a photosensitive radical reaction. In the present invention, it has been proven that such structures on surface-bonded continuous copolymer layers of the invention enhance the capturing and separation of agents and/or objects, in particular cells. It has surprisingly been found that the present invention reduces non-specific background reactions and allows for an increase in speed, specificity and sensitivity of the detection of analytes. Accordingly, the means and methods provided by the present invention are particularly useful in analytical and/or diagnostic applications, including immunoaffinity assays, ELISA or ELISpot assay.
In certain aspects of the invention, the copolymer is characterized by a monolithic, topographical modification of the surface through a unique, temporally and spatially selective exposure to UV light. After this exposure, either a UV-reactive side chain or the click-reactive side chain is retained to bind a biomolecule. The surface can be made up of polystyrenes, cyclic olefin polymers, poly(methyl methacrylates) or polycarbonates. The hydrogel layer can be used topographically for the separation of objects, in particular cells. For example, circular column structures (diameter and/or width 10-50 µm, height 1-10 µm) with circular distances (diameter 10-50 µm) in a hexagonal arrangement can be used. For the production of the hydrogel layer, the polymer can be dissolved in a solvent, in particular ethanol, and dispensed, e.g., into a container. After the ethanol has evaporated, the polymer layer can be cross-linked to the hydrogel by means of UV light (e.g., 315-380 nm) and attached to the surface of the bottom end of the container. If the polymer offers a bond-reactive group (e.g., azide), a previously modified biomolecule (e.g., an antibody which has been labeled with an alkaline group, e.g., dibenzocyclooctyne (DBCO)) can be coupled to it. This biomolecule can then bind the analyte that is to be detected in, e.g., an immunoaffinity assay. The analyte can then be bound to another epitope in these assays with a second enzyme-labeled antibody. In a further step, the enzyme can then initiate a color reaction on the substrate.

In one aspect, the present invention provides a method of manufacturing a surface-bonded continuous layer of a copolymer with a synthetic one-component system comprising at least one photo-reactive side group for a C, H-insertion cross-linking ["CHic"] reaction; and at least one click binding-reactive side group ["Click"] for (bio-)functionalization of the copolymer layer. In a second aspect, the invention provides a method of manufacturing a container having a surface-bonded continuous layer of such a copolymer with a synthetic one-component system comprising at least one photo-reactive side group for a C, H-insertion cross-linking ["CHic"] reaction; and at least one click binding-reactive side group ["Click"] for (bio-) functionalization of the copolymer layer. In a third aspect, the invention provides methods of 3D functionalization of a container using the surface-bonded continuous layer of a copolymer of the invention. In a fourth aspect, the invention provides a surface-bonded continuous layer of a copolymer manufactured by the methods disclosed herein. In a fifth aspect, the invention provides a container manufactured by the methods disclosed herein. In a sixth aspect, the invention provides methods of using the manufactured container in analytical and/or diagnostic applications.

The present invention provides in particular:
[1] A method of manufacturing a surface-bonded continuous layer of a copolymer, comprising the steps:
   (a) providing a copolymer dissolved in a solvent, wherein the copolymer comprises at least three different monomer units and includes
      (i) at least one photo-reactive side group for a C, H-insertion cross-linking ["CHic"] reaction; and
      (ii) at least one click binding-reactive side group ["Click"] for (bio-)functionalizing of the copolymer layer;
   (b) applying the dissolved copolymer of (a) to a surface and forming a continuous copolymer layer on the said surface;
   (c) after evaporation of the solvent, exposing the continuous copolymer layer of (b) to UV light for cross-linking [of the copolymer (chains)] and binding [of the cross-linked copolymer] to the said surface; and optionally
   (d) obtaining a surface-bonded continuous layer of a copolymer.
[2] A method of manufacturing a container having a continuous layer of a copolymer bonded to the surface of the bottom end of the container, comprising the steps:
   (a) providing a copolymer solved in a solvent, wherein the copolymer comprises at least three different monomer units and includes
      (i) at least one photo-reactive side group for a C, H-insertion cross-linking ["CHic"] reaction; and
      (ii) at least one click binding-reactive side group ["Click"] for (bio-) functionalizing of the copolymer layer;
   (b) applying the dissolved copolymer of (a) to the surface of the bottom end of a container and forming a continuous copolymer layer on the said surface;
   (c) after evaporation of the solvent, exposing the continuous copolymer layer of (b) to UV light for cross-linking and binding to the said surface; and optionally
   (d) obtaining a container having a continuous layer of a copolymer bonded to the surface of the bottom end of the container.
[3] The method of [2], wherein the container is formed of polymeric material and has an open top end, a closed bottom end, and at least one wall extending between said ends.
[4] The method of any one of [1] to [3], wherein the amount of copolymer applied to the surface is at least 0.5 µg/mm², preferably at least 8-10 µg/mm².
[5] The method of any one of [1] to [4], further comprising a step of photostructuring the surface-bonded copolymer layer by a spatially selective exposure of the copolymer layer to light, preferably UV light.
[6] The method of [5], wherein the photostructuring generates hill-like structures on the continuous copolymer layer while maintaining a monolithic topography, preferably wherein the hill-like structures exhibit a hexagonal arrangement.
[7] The method of [5] or [6], wherein the hill-like structures have a diameter of 10-50 µm and/or a height of 1-10 µm.
[8] A surface-bonded continuous layer of a copolymer obtainable by the method of any one of [1] and [4] to [7].
[9] A container having a continuous layer of a copolymer bonded to the surface of the bottom end of the container obtainable by the method of any one of [2] to [7].
[10] A container formed of polymeric material and having an open top end, a closed bottom end, and at least one wall extending between said ends, characterized in that the surface of the bottom end has a surface-bonded continuous layer of a copolymer comprising at least three different monomer units and including
   (i) at least one photo-reactive side group for a C, H-insertion cross-linking ["CHic"] reaction; and
   (ii) at least one click binding-reactive side group ["Click"] for (bio-)functionalizing of the copolymer layer.
[11] The container of [10], wherein the copolymer layer is photostructured while maintaining a monolithic topography, preferably wherein the copolymer layer has hill-like structures, preferably wherein the hill-like structures exhibit a hexagonal arrangement.
[12] The method of any one of claims [2] to [7], or the container of any one of [9] to [11], wherein the container comprises an optically transparent part, and/or wherein the container has an essentially tubular shape and/or has a conical shape towards the bottom end of the container, preferably wherein the container is a laboratory test tube, e.g. a well of a microplate.
[13] The method of any one of [1] to [7], or the surface-bonded continuous layer of a copolymer of [8], or the container of any one of [9] to [12], wherein the at least three different monomer units comprise a hydrophilic monomer, preferably a hydrophilic acryl monomer, and/or wherein at least one monomer is a photoreactive crosslinker, and/or wherein the at least one click binding-reactive side group is an azide or a derivative thereof.
[14] A container assembly, or an analytical device, comprising one or more of a surface-bonded continuous layer of a copolymer according to [8], or one or more of a container according to any one of [9] to [13].
[15] Use of the surface-bonded continuous layer of a copolymer according to [8] or [20], or the container of any one of [9] to [13] and [21], or the container assembly or analytical device of [14] or [22], in analytical and/or diagnostic applications, preferably for use in an immunoaffinity assay, ELISA or ELISpot assay.
[16] The use of [15], which is for separation of dispersed particles and/or objects, preferably wherein the dispersed particles and/or objects are cells.
[17] The use of [15], which is for the selective covalent attachment and/or capturing of biochemical reagents to the copolymer layer.
[18] A method of separating dispersed particles and/or objects, comprising a surface-bonded continuous layer of a copolymer according to [8], or the container of any one of [9] to [13], or the container assembly or analytical device of [14], preferably wherein the dispersed particles and/or objects are cells.
[19] The method of any one of [1] to [7], wherein step (a) comprises:
   providing a copolymer dissolved in a solvent, wherein the copolymer comprises at least three different monomer units and includes
   (i) at least one photo-reactive side group for a C, H-insertion cross-linking ["CHic"] reaction;
   (ii) at least one click binding-reactive side group ["Click"] for (bio-) functionalizing of the copolymer layer; and
   (iii) a hydrophilic monomer, preferably a hydrophilic acryl monomer;
   and/or wherein at least one monomer is a photoreactive crosslinker, and/or wherein the at least one click binding-reactive side group is an azide or a derivative thereof.
[20] A surface-bonded continuous layer of a copolymer obtainable by the method of [19].
[21] The container of any one of [9] to [13], characterized in that the surface of the bottom end has a surface-bonded continuous layer of a copolymer comprising at least three different monomer units and including
   (i) at least one photo-reactive side group for a C, H-insertion cross-linking ["CHic"] reaction;
   (ii) at least one click binding-reactive side group ["Click"] for (bio-)functionalizing of the copolymer layer, and
   (iii) a hydrophilic monomer, preferably a hydrophilic acryl monomer;
   and/or wherein at least one monomer is a photoreactive crosslinker, and/or wherein the at least one click binding-reactive side group is an azide or a derivative thereof.
[22] A container assembly, or an analytical device, comprising one or more of a surface-bonded continuous layer of a copolymer according to [8], or one or more of a container according to [21].

### BRIEF DESCRIPTION OF THE DRAWINGS

**Figure 1****:** Example of a structural formula for a synthetic one-component compound.
**Figure 2****:** Schematic representation of an exemplary coating of microtiter plates using a manual dispensing process.
**Figure 3****:** Light microscope images of a plastic surface, modified by a synthetic one-component connection, whereby monolithic topographies with circular column structures were created. The uptake was carried out **(A)** in a dry state and **(B)** in a state swollen with PBS buffer.
**Figure 4****:** Measurement of the monolithic topography with column structure using an atomic force microscope [Atomic Force Microscopy (AFM)]. **(A)** 2D AFM image of a dry structured coating with a corresponding height profile (profil1) **(B)** The associated 3D AFM image showing the column structure.
**Figure 5****:** Height profile of a structured coating measured with a mechanical profilometer. The smaller the distance between the exposed areas, the more copolymer is also crosslinked at those areas that are not exposed to direct UV light.
**Figure 6****:** Binding capacity measurements of different coatings: Streptavidin was first covalently bound using click chemistry. The detection is then carried out by means of fluorescence-labeled Atto 550-Biotin (Sigma, #28923) using supernatant measurements.
**Figure 7****:** Light microscope images to investigate the mechanical stability using strong **(A and B)** and light **(C and D)** scraping with a pipette tip: the coating (A and C) is compared to an ELISpot filter base plate on the market (B and D) only slightly damaged. In the case of the reference plate, the plate floor even breaks through when scratched vigorously (B).
**Figure 8****:** Light microscope image of a separation of polystyrene particles (Ø≈7 µm) on the monolithic topography.
**Figure 9****:** Light microscope images during the sedimentation process of "mononuclear cells of the peripheral blood". Peripheral Blood Mononuclear Cell (PBMC)]. Shots are shown after certain time intervals. The arrows highlight cells that first hit a column and then migrate into a micro-pocket.
**Figure 10****:** Light microscope images of PBMC's after incubation overnight in the ELISpot test. **(A)** Un-structured surface. **(B)** Structured surface.
**Figure 11****:** Light microscope images of representative ELISpot tests (stimulus: 30 µg / ml PHA-M) for **(A)** a reference plate **(B)** and plates coated with the one-component copolymer without microstructures and **(C)** with microstructures. More and smaller spots can be seen on the coated plates compared to the reference plate, which shows fewer and larger spots.
**Figure 12****:** Light microscope images of the ELISpot test with a structured surface. The enlargements always show precisely defined spots between 3 columns. Exactly one cell always sedimented at these points.
**Figure 13****:** IL-6 ELISA on a hydrogel plate.
**Figure 14****:** Binding capacity test with Atto 590-Biotin on a hydrogel plate.

### DETAILED DESCRIPTION OF THE INVENTION

In one aspect, the invention provides a method of manufacturing a surface-bonded continuous layer of a copolymer with a synthetic one-component system comprising at least one photo-reactive side group for a C, H-insertion cross-linking ["CHic"] reaction; and at least one click binding-reactive side group ["Click"] for (bio-)functionalizing of the copolymer layer. In particular, the present invention provides a method of manufacturing a surface-bonded continuous layer of a copolymer, comprising the steps:
(a) providing a copolymer dissolved in a solvent, wherein the copolymer comprises at least three different monomer units and includes
   (i) at least one photo-reactive side group for a C, H-insertion cross-linking ["CHic"] reaction; and
   (ii) at least one click binding-reactive side group ["Click"] for (bio-) functionalizing of the copolymer layer;
(b) applying the dissolved copolymer of (a) to a surface and forming a continuous copolymer layer on the said surface;
(c) after evaporation of the solvent, exposing the continuous copolymer layer of (b) to UV light for cross-linking [of the copolymer (chains)] and binding [of the cross-linked copolymer] to the said surface.

In various embodiments, the method involves obtaining a surface-bonded continuous layer of a copolymer subsequent to steps (a), (b), and (c).

As used herein, the term "copolymer" preferably refers to alternating polymers formed by the polymerization reaction of at least two different monomers, i.e., two, three or more different monomers. Thus, the term "copolymer" as used herein does not exclude, e.g., so-called terpolymers obtained from co-polymerization of three different monomers. The term "alternating copolymer" as used herein refers to a polymer having two or more different types of monomers joined together in the same polymer chain wherein the different monomers are arranged in a random (statistical) order.

In various preferred embodiments, the copolymer is a one-component compound, preferably wherein the copolymer is an alternating copolymer. A component as used herein may be one of a collection of chemically-independent constituents of a system. The number of components represents the minimum number of independent species necessary to define the composition of all phases of the system. Accordingly, one-component compound as used herein means that the copolymer forming part of the method of the invention represents a compound that requires only one of the independent monomers of the copolymer in order to define all phases of the compound. Accordingly, the term "one-component system" means that all of the components of the copolymer which are required for the subsequent steps are already contained in the one-component copolymer. The term "monomer" as used herein can refer to a chemical (or material thereof) characterized as having a molecule (or unit) that can bind to other molecules. Large numbers of monomer units can bind to form polymers.

In various embodiments, the copolymer forming part of the method of the invention comprises at least one monomer unit that is a photo-reactive side group for a C, H-insertion cross-linking reaction. In preferred embodiments, the photo-reactive side group for a C, H-insertion cross-linking reaction is a photoreactive crosslinker. C, H-insertion reactions concern the insertion reaction of e.g. carbenes, nitrenes or ketyl biradicals into a carbon-hydrogen bond. This organic reaction is of some importance in the synthesis of new organic compounds in which only one reactive group is contained, which after activation reacts with the neighboring polymer chains through C, H-insertion reactions. In such reactions the cross-linking units can be incorporated into the polymer matrix and induce the linking of the involved polymer chains after thermal or UV-activation by (formal) C, H-insertion. Upon imparting heat or light into the film, the cross-linker units can be activated and generate a reactive intermediate, which depending on the chemical nature of cross-linker reacts with almost any groups in the neighboring chains through a C, H-insertion reaction (CHic = C, H-insertion based crosslinking). This crosslinking reaction pathway offers the advantage that there is no need that two reaction partners meet each other before connecting as one reaction partner - the C, H group - is present in abundance. The term "photoreactive crosslinker" refers to photo-activable reactive groups for binding proteins, nucleic acids and other biomolecules in an irreversible manner after activated by ultraviolet or visible light. Examples of photoreactive crosslinkers include, but are not limited to, arylazides, azido-methylcoumarins, benzophenones, anthraquinones, certain diazo compounds, diazirines, and psoralen derivatives. In preferred embodiments, the photoreactive crosslinker used in the present invention is methacryloyloxybenzophenone (MABP). In other preferred embodiments, the copolymer is an alternating copolymer. In still other preferred embodiments, the copolymer is a one-component compound. In further preferred embodiments, the copolymer is a one-component compound, preferably wherein the copolymer is an alternating copolymer.

The copolymer forming part of the method of the invention comprises at least one monomer unit that is a click binding-reactive side group ["Click"]. Binding-reactive side group may include for example, chemically reactive groups, biologically reactive groups or bioaffinity agents. The term "Click" as used herein generally refers to 'click chemistry', i.e., any bioorthogonal reaction that enables the efficient formation of a specific product within a highly complex chemical environment. In particular, it refers to a chemical reaction which allows for the incorporation of one or more reactive tag(s) onto a biomolecular target and the subsequent high selectivity in tag derivatization within a complex biological sample. Click chemistry results in the formation of such substances quickly and reliably. That is, the reactions are efficient, high yielding, and tolerant of various solvents and functional groups. In various embodiments, click binding reaction includes, without being limited thereto, nucleophilic substitutions, particularly to a small strained ring (e.g., epoxides, aziridines, aziridinium ions, and episulfonium ions), addition to carbon-carbon double or triple bonds (e.g., thiol-ene, tiol-yne reactions, dihydroxilation, epoxidation, and aziridination), cycloadditions (e.g., [3+2] cycloadditions, [4+1] cycloadditions, and Diels-Alder), "non-aldol"-type carbonyl chemistry (e.g., formation of ureas, thioureas, aromatic heterocycles, oxime ethers, hydrazones, and amides). Accordingly, in various embodiments, the at least one click binding-reactive side group ["Click"] for (bio-) functionalizing of the copolymer layer may provide for, or effect, a click binding reaction of any one of nucleophilic substitutions, particularly to a small strained ring (e.g., epoxides, aziridines, aziridinium ions, and episulfonium ions), addition to carbon-carbon double or triple bonds (e.g., thiol-ene, tiol-yne reactions, dihydroxilation, epoxidation, and aziridination), cycloadditions (e.g., [3+2] cycloadditions, [4+1] cycloadditions, and Diels-Alder), and "non-aldol"-type carbonyl chemistry (e.g., formation of ureas, thioureas, aromatic heterocycles, oxime ethers, hydrazones, and amides). In various embodiments, cycloadditions are preferred, more preferably [3+2] cycloadditions, even more preferably 1,3 dipolar additions. In various embodiments, cycloaddition with an azide (group), or a derivative thereof, is preferred.

The term 'biofunctionalization' of the copolymer as used herein refers to the formation of click binding-reactive groups on the copolymer for modulating the interaction and/or immobilization of biomolecules. In preferred embodiments, the click binding-reactive side group ["Click"] used in the invention is an azide or its derivative thereof. The term "azide" as used herein refers to any compound having the N moiety therein. The azide may be a metal azide wherein the metal is an alkali metal such as potassium, sodium, lithium, rubidium or cesium. The metal may be a transition metal such as, but not limited to, iron, cobalt, nickel, copper or zinc. It is understood that certain metallic azides may be formed in solution by mixing sodium azide or the like with a metallic salt such as, for example, copper sulfate. The azide of the present invention may also be an organic azide or ammonium azide. In various preferred embodiments, the azide is N-(3-azidopropyl)methacrylamide (AzMA). In various preferred embodiments, the copolymer is an alternating copolymer. In other preferred embodiments, the copolymer is a one-component compound. In further preferred embodiments, the copolymer is a one-component compound, preferably wherein the copolymer is an alternating copolymer.

In other embodiments, the copolymer forming part of the method of the invention comprises a hydrophilic monomer unit with or without partial charge. The hydrophilic monomer unit with or without partial charge includes hydrophilic acryl monomers, preferably acrylamides and its derivatives. The term acrylamide as used herein includes unsubstituted acrylamide and derivatives thereof, such as methacrylamide, and the like, as well as N-substituted derivatives thereof. In preferred embodiments, the acrylamide as used herein is dimethylacrylamide (DMAA). In other preferred embodiments, the copolymer is an alternating copolymer. In other preferred embodiments, the copolymer is a one-component compound. In further preferred embodiments, the copolymer is a one-component compound, preferably wherein the copolymer is an alternating copolymer.

The copolymer forming part of the method of the invention comprises at least one monomer unit that is a photo-reactive side group for a C, H-insertion cross-linking reaction, preferably a photoreactive cross linker, and more preferably a methacryloyloxybenzophenone (MABP), and at least one monomer unit is a click binding-reactive side group ["Click"], preferably an azide or its derivatives, more preferably N-(3-azidopropyl)methacrylamide (AzMA). In various preferred embodiments, the copolymer is an alternating copolymer. In other preferred embodiments, the copolymer is a one-component compound. In further preferred embodiments, the copolymer is a one-component compound, preferably wherein the copolymer is an alternating copolymer.

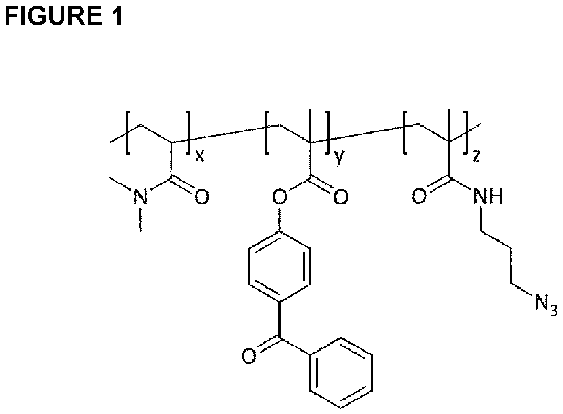

The copolymer forming part of the method of the invention comprises three monomer units, as exemplified in Figure 1 and explained in Example 1. In various embodiments, the copolymer comprises at least one monomer unit that is a photo-reactive side group for a C, H-insertion cross-linking reaction, preferably a photoreactive cross linker and more preferably a methacryloyloxybenzophenone (MABP), and at least one monomer unit that is a click binding-reactive side group ["Click"], preferably an azide or its derivatives, more preferably N-(3-azidopropyl)methacrylamide (AzMA), and a hydrophilic monomer unit with or without partial charge, including hydrophilic acryl monomers, preferably, e.g., acrylamides and its derivatives, more preferably dimethylacrylamide (DMAA). In preferred embodiments, the copolymer is an alternating copolymer. In other preferred embodiments, the copolymer is a one-component compound. In further preferred embodiments, the copolymer is a one-component compound, preferably wherein the copolymer is an alternating copolymer.

Figure 1 exemplifies the proportion of individual monomer units in percentages used in the method of manufacturing the copolymer of the invention according to any of the embodiments mentioned above. DMAA is represented by x%, MABP as y% and AZMA as z%. In various embodiments, the proportion of the hydrophilic monomer unit with or without partial charge, including hydrophilic acryl monomers, preferably acrylamides and its derivatives, preferably dimethylacrylamide (DMAA), is between 88% to 98%, preferably between 87.5% and 97.5%, between 89% and 96%, between 90% and 95%, between 91% and 94%, between 92% and 93%, preferably 87.5%, or 89%, or 92%, or 90%, and more preferably 94%. Each alternative represents a separate embodiment of the invention. In other embodiments, the proportion of the at least one monomer unit that is a photo-reactive side group for a C, H-insertion cross-linking reaction, including a photoreactive cross linker, preferably a methacryloyloxybenzophenone (MABP), is between 2% and 5%, between 2.5% and 4.5%, between 3.5% and 4%, preferably between 2% and 3%, and most preferably 2.5%. Each alternative represents a separate embodiment of the invention. In other embodiments, the proportion of the at least one monomer unit that is a click binding-reactive side group ["Click"], including an azide or its derivatives, preferably N-(3-azidopropyl)methacrylamide (AzMA), is between 3% and 9%, preferably 3.5%, or 5.5% or 7.5% or 8.5%, and more preferably 3.5%. According to the method of manufacturing the copolymer layer described according to the embodiments mentioned herein above, the copolymer according to the embodiments described herein above is dissolved in a suitable solvent for the production of the hydrogel layer. The term "solvent" as used herein refers to an organic solvent, a mixture of organic solvents, an organic solvent or mixture of organic solvents and water containing salts or no salts buffered or non-buffered. "Organic solvent" refers to, e.g., hydrocarbons such as, e.g., hexane, cyclo-hexane, heptane, toluene, xylenes, ketones such as, e.g., tert-butyl methyl ketone, methyl isopropyl ketone, 2-butanone, acetone, 4-methyl-2-pentanone, ethers such as, e.g., diethylether, tert-butyl methyl ether (MTBE), isopropyl methyl ether, dioxane, dibutylether, dioxolane, anisole, and tetrahydrofuran (THF), nitriles such as, e.g., acetonitrile and 3-hydroxypropionitrile, polar solvents such as, e.g., dimethyl sulfoxide (DMSO), sulfolane, N,N'-dimethylpropyleneurea (DMPU), glyoxal, acids such as, e.g., acetic acid and formic acid, aldehydes such as, e.g., acetaldehyde, halogenated hydrocarbons such as, e.g., dichloromethane (DCM), trichloroethane, chloroform, chloroben-zene, dichlorobenzene, and dichloroethane, esters such as, e.g., ethyl acetate (EtOAc), isopropyl acetate, or tert-butyl acetate, straight or branched alcohols such as, e.g., 2-methyl-2-butanol, tert-butanol, methanol, ethanol, n-propanol, n-butanol, and 2-propanol. In other preferred embodiments, the term "solvent" as used herein refers to buffered (e.g., phosphate, acetate), non-buffered water, or buffered or non-buffered water containing a water miscible organic solvent such as acetone, tetrahydrofuran (THF), 2-propanol, ethanol, t-butanol, dimethylformamide, dimethyl sulfoxide, or 2-methyl-2-pentanone or ethers, such as tert-butylmethyl ether (MTBE), saturated or not saturated with water. In preferred embodiments, the solvent for the production of the hydrogel is ethanol, or 2-propanol. The dissolved copolymer in the solvent may be applied to a surface at an amount of at least 0.5 µg/mm² or 16 µg/well to at least 8-10 µg/mm² or 300 µg/well. In the next step, the surface is heated to a temperature range between 35 to 70°C, preferably 40 to 70°C, 45 to 70°C, 50 to 70°C, 55 to 70°C, 60 to 70°C, and 65 to 70°C, even more preferably, 35°C, or 45°C or 47°C, or 53°C or 70°C.

In preferred embodiments, according to the method of manufacturing a surface-bonded continuous layer of a copolymer of the invention, the cross-linking of the copolymer and surface bond is achieved by a radical reaction using UV-light. As used herein, the term "surface bonded" refers to a substituent that is substantially bonded, either covalently or ionically, primarily or only to the outer surface of a material. The term "surface" refers to a solid surface, i.e., a material having a rigid or semi-rigid surface. As used herein, the method of manufacturing a surface-bonded continuous layer of a copolymer includes providing a surface on which the copolymer layer is bonded. Surface as used herein is preferably a polymeric material. The polymeric material preferably consists of polystyrenes, cyclic olefin polymers, poly(methyl methacrylates) or polycarbonates.

In preferred embodiments, the surface-bond is a covalent bond. This means that there is no reaction equilibrium but an irreversible binding of the synthetic copolymer of the invention to a surface. In further preferred embodiments, the radical reaction takes place between the monomer unit of the synthetic copolymer that is a photo-reactive side group and the surface. In various embodiments of the invention, the copolymer layer is surface-bonded by a selective UV exposure. In preferred embodiments, the selective UV exposure is spatial and temporal. The selective UV exposure allows the copolymer to form a cross-linked copolymer and allows covalent bonding to the surface in order to produce a monolithic coating via the photo-reactive side group of a photoreactive crosslinker described herein for a C, H-insertion cross-linking reaction. In preferred embodiments, the UV exposure is at a wavelength of between 315 to 380 nm, preferably 365 nm and the energy of 4 J/cm². The copolymer layer can be surface-bonded and crosslinked by a single exposure, the exposure duration or the energy input plays an important role and also influences the properties of the coating in particular the number of crosslinking points and degree of swelling of the layer.

In preferred embodiments, the degree of swelling is between 1.5 and 15, preferably between 2 and 4. The term "degree of swelling" is defined as follows: ((volume of crosslinked copolymer) + (volume of a compound used in swelling)) / (volume of crosslinked copolymer). The term "volume of a compound (solvent) used in swelling" refers to the volume of a compound (solvent) accumulated in the crosslinked copolymer.

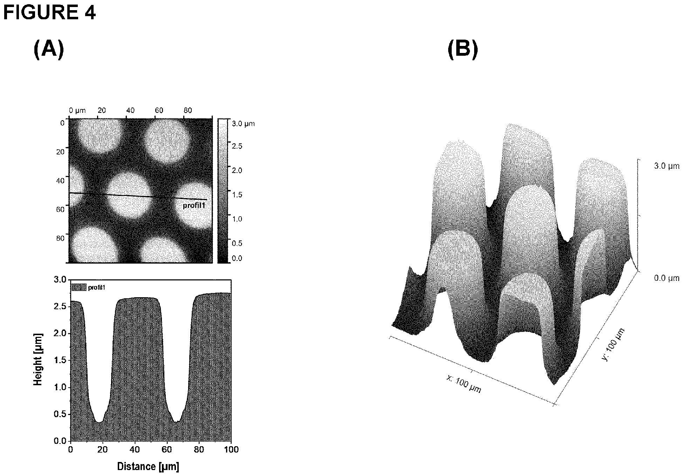

In preferred embodiments, the method of manufacturing a surface-bonded copolymer layer of the invention provides for a continuous layer. This is demonstrated in Figure 4 that confirms that the cross-linked copolymer is also present between the hills. The term "continuous" as used herein means a coating of the surface-bonded layer of copolymer having an uninterrupted extension in along a surface. Other ways to describe continuously include but are not limited to "constantly" or "reoccurring in rapid succession". Accordingly, as used herein, the term "continuous" refers to a layer that covers an entire surface without gaps or bare spots that reveal material underlying the layer. The term "continuous" as used herein is meant to encompass the surface-bonded layer of copolymer having unintentional, intermittent, isolated interruptions or gaps (e.g., from a manufacturing defect) from a discontinuous deposit. These gaps or bare spots on the surface may be less than about 1 % of the total surface area. Accordingly, a preferred embodiment of the invention regarding the method of manufacturing a surface-bonded, continuous copolymer layer comprises creating a solution from a single copolymer using a solvent, wherein the solvents preferably consisting of alcoholic solutions, in particular ethanol and 2-propanol, but also acetonitrile or water, or aqueous mixtures with other solvents; and the dissolved copolymer is applied to the surface preferably by using spraying and dispensing techniques; wherein the applied mass of the copolymer is at least 0.5 µg/mm² or 16 µg/well to at least 8-10 µg/mm² or 300 µg/well; and the wetting of the surface is controlled by the temperature and/or concentration of the polymer solution; and after evaporation of the solvent, the copolymer layer is cross-linked by means of UV light, preferably UV-A (315-380 nm), and bound to the surface.

The present invention also provides a method of manufacturing a container having a surface-bonded continuous layer of a copolymer with a synthetic one-component system comprising at least one photo-reactive side group for a C, H-insertion cross-linking ["CHic"] reaction; and at least one click binding-reactive side group ["Click"] for (bio-) functionalizing of the copolymer layer. In particular, the present invention provides a method of manufacturing a container having a continuous layer of a copolymer bonded to the surface of the bottom end of the container, comprising the steps:
(a) providing a copolymer solved in a solvent, wherein the copolymer comprises at least three different monomer units and includes
   (i) at least one photo-reactive side group for a C, H-insertion cross-linking ["CHic"] reaction; and
   (ii) at least one click binding-reactive side group ["Click"] for (bio-) functionalizing of the copolymer layer;
(b) applying the dissolved copolymer of (a) to the surface of the bottom end of a container and forming a continuous copolymer layer on the said surface;
(c) after evaporation of the solvent, exposing the continuous copolymer layer of (b) to UV light for cross-linking and binding to the said surface.

In various embodiments, the method involves obtaining a container having a continuous layer of a copolymer bonded to the surface of the bottom end of the container subsequent to steps (a), (b), and (c). The container may be formed of polymeric material and has an open top end, a closed bottom end, and at least one wall extending between said ends. In various embodiments, the amount of copolymer applied to the surface is at least 0.5 µg/mm², preferably at least 8-10 µg/mm².

As used herein, the term "container" refers to any hollow rigid object suitable for supporting and containing a product. The container is optionally provided with a sealing area around the perimeter of the opening of the container, a rim or at least around the perimeter of the opening of the container, such as a sealing flange, suitable for being sealed to the heat sealable layer of a film forming the lid of the container. The containers of the present invention are suitable for use as a container for any kind of matter, but preferably for liquids or other compositions which are fluid. The term container does not imply a particular intended use for the article. For example, the term "container" as used herein encompasses articles destined to comprise a surface-bonded continuous layer of a copolymer of the invention. As used herein the term "container" may also be interpreted as "well". The term "well" as used herein is a sample holder such as a small test tube where a biological or other sample is placed in microtiter plate. Accordingly, the term 'container assembly' or 'analytical device' according to the invention may refer to microtiter plates. "Microtiter plates" (also referred to as microplates and microwell plates) generally are a substantially flat plate with multiple wells arranged in an array. Microtiter plates can be configured with from about 6 to about 9600 wells. Microtiter plates have multiple uses including but not limited to holding and transporting liquids, performing biological or chemical reactions, combinations thereof and the like. Microtiter plates frequently are used in research or diagnostic procedures including high throughput protocols.

The container having a surface-bonded continuous layer of a copolymer forming part of the invention is characterized in that the copolymer comprises at least one monomer unit that is a photo-reactive side group for a C, H-insertion cross-linking reaction. In preferred embodiments, the photo-reactive side group for a C, H-insertion cross-linking reaction is a photoreactive crosslinker. C, H-insertion reactions concern the insertion reaction of, e.g., carbenes, nitrenes or ketyl biradicals into a carbon-hydrogen bond. This organic reaction is of some importance in the synthesis of new organic compounds in which only one reactive group is contained, which after activation reacts with the neighboring polymer chains through C, H-insertion reactions. In such reactions the cross-linking units can be incorporated into the polymer matrix and induce the linking of the involved polymer chains after thermal or UV-activation by (formal) C, H-insertion. Upon imparting heat or light into the film, the cross-linker units can be activated and generate a reactive intermediate, which depending on the chemical nature of cross-linker reacts with almost any groups in the neighboring chains through a C, H-insertion reaction (CHic = C, H-insertion based crosslinking). This crosslinking reaction pathway offers the advantage that there is no need that two reaction partners meet each other before connecting as one reaction partner - the C, H group - is present in abundance. The term "photoreactive crosslinker" refers to photo-activable reactive groups for binding proteins, nucleic acids and other biomolecules in an irreversible manner after activated by ultraviolet or visible light. Examples of photoreactive crosslinkers include, but are not limited to, arylazides, azido-methyl-coumarins, benzophenones, anthraquinones, certain diazo compounds, diazirines, and psoralen derivatives. In a preferred embodiment, the photoreactive crosslinker used in the present invention is methacryloyloxybenzophenone (MABP). In preferred embodiments, the copolymer is an alternating copolymer. In other preferred embodiments, the copolymer is a one-component compound. In further preferred embodiments, the copolymer is a one-component compound, preferably wherein the copolymer is an alternating copolymer. The container having a surface-bonded continuous layer of a copolymer forming part of the invention is further characterized in that the copolymer comprises at least one monomer unit that is a click binding-reactive side group ["Click"]. Binding-reactive side group may include for example, chemically reactive groups, biologically reactive groups or bioaffinity agents. The term "click" as used herein generally refers to 'click chemistry' i.e. any bioorthogonal reaction that enables the efficient formation of a specific product within a highly complex chemical environment. In particular, it refers to a chemical reaction which allows for the incorporation of one or more reactive tag(s) onto a biomolecular target and the subsequent high selectivity in tag derivatization within a complex biological sample. Click chemistry results in the formation of such substances quickly and reliably. That is, the reactions are efficient, high yielding, and tolerant of various solvents and functional groups. The term 'biofunctionalization' of the copolymer as used herein refers to the formation of click binding-reactive groups on the copolymer for modulating the interaction and/or immobilization of biomolecules. In preferred embodiments, the click binding-reactive side group ["Click"] used in the invention is an azide or its derivative thereof. The term "azide" as used herein refers to any compound having the N moiety therein. The azide may be a metal azide wherein the metal is an alkali metal such as potassium, sodium, lithium, rubidium or cesium. The metal may be a transition metal such as, but not limited to, iron, cobalt, nickel, copper or zinc. It is understood that certain metallic azides may be formed in solution by mixing sodium azide or the like with a metallic salt such as, for example, copper sulfate. The azide of the present invention may also be an organic azide or ammonium azide. In preferred embodiments, the azide is N-(3-azidopropyl)methacrylamide (AzMA). In other preferred embodiments, the copolymer is an alternating copolymer. In further preferred embodiments, the copolymer is a one-component compound. In still further preferred embodiments, the copolymer is a one-component compound, preferably wherein the copolymer is an alternating copolymer.

In various embodiments, the container having a surface-bonded continuous layer of a copolymer is characterized in that the copolymer comprises a hydrophilic monomer unit with or without partial charge. The hydrophilic monomer unit with or without partial charge includes hydrophilic acryl monomers, preferably acrylamides and its derivatives. The term acrylamide as used herein includes unsubstituted acrylamide and derivatives thereof, such as methacrylamide, and the like, as well as N-substituted derivatives thereof. In preferred embodiments, the acrylamide as used herein is dimethylacrylamide (DMAA). In preferred embodiments, the copolymer is an alternating copolymer. In other preferred embodiments, the copolymer is a one-component compound. In further preferred embodiments, the copolymer is a one-component compound, preferably wherein the copolymer is an alternating copolymer. In preferred embodiments, the container having a surface-bonded continuous layer of a copolymer forming part of the method of the invention is characterized in that the copolymer comprises at least one monomer unit that is a photoreactive cross linker, preferably a methacryloyloxybenzophenone (MABP), and an azide or its derivatives, more preferably N-(3-azidopropyl)methacrylamide (AzMA) as at least one monomer unit providing for the click binding-reactive side group ["Click"]. In preferred embodiments, the copolymer is an alternating copolymer. In other preferred embodiments, the copolymer is a one-component compound. In further preferred embodiments, the copolymer is a one-component compound, preferably wherein the copolymer is an alternating copolymer.

In preferred embodiments, the container having a surface-bonded continuous layer of a copolymer forming part of the method of the invention is characterized in that the copolymer comprises three monomer units as shown in Figure 1 and explained in Example 1. In particular, the copolymer comprises at least one monomer unit that is a photo-reactive side group for a C, H-insertion cross-linking reaction, preferably a photoreactive cross linker and more preferably a methacryloyloxybenzophenone (MABP), and at least one monomer unit that is a click binding-reactive side group ["Click"], preferably an azide or its derivatives, more preferably N-(3-azidopropyl)methacrylamide (AzMA), and a hydrophilic monomer unit with or without partial charge, including hydrophilic acryl monomers, preferably acrylamides and its derivatives, preferably dimethylacrylamide (DMAA). In preferred embodiments, the copolymer is an alternating copolymer. In other preferred embodiments, the copolymer is a one-component compound. In further preferred embodiments, the copolymer is a one-component compound, preferably wherein the copolymer is an alternating copolymer.

Figure 1 exemplifies the proportion of individual monomer units in percentages that can be used in the method of manufacturing the container having a surface-bonded continuous layer of a copolymer of the invention according to any of the embodiments disclosed herein. DMAA is represented by x%, MABP as y% and AZMA as z%. In various embodiments, the composition of the hydrophilic monomer unit with or without partial charge, including hydrophilic acryl monomers, preferably acrylamides and its derivatives, preferably dimethylacrylamide (DMAA), is between 88% to 98%, preferably between 87.5% and 97.5%, between 89% and 96%, between 90% and 95%, between 91% and 94%, between 92% and 93%, preferably 87.5%, or 89%, or 92%, or 90%, and more preferably 94%. Each alternative represents a separate embodiment of the invention. In other embodiments, the composition of the at least one monomer unit that is a photo-reactive side group for a C, H-insertion cross-linking reaction, preferably a photoreactive cross linker, and more preferably a methacryloyloxybenzophenone (MABP) is between 2% and 5%, between 2.5% and 4.5%, between 3.5% and 4%, preferably between 2% and 3%, and more preferably 2.5%. Each alternative represents a separate embodiment of the invention. In further embodiments, the composition of at least one monomer unit that is a click binding-reactive side group ["Click"], preferably an azide or its derivatives, more preferably N-(3-azidopropyl)methacrylamide (AzMA), is between 3% and 9%, preferably 3.5%, or 5.5% or 7.5% or 8.5%, and more preferably 3.5%.

The methods disclosed herein may further comprise a step of photostructuring the surface-bonded copolymer layer by a spatially selective exposure of the copolymer layer to light, preferably UV light. In various embodiments, the photostructuring generates hill-like structures on the continuous copolymer layer while maintaining a monolithic topography, preferably wherein the hill-like structures exhibit a hexagonal arrangement. In various preferred embodiments, the hill-like structures have a diameter and/or width of 10-50 µm and/or a height of 1-10 µm.

In various embodiments, the selective UV exposure involves the use of a photomask in order to photostructure the surface-bonded copolymer layer. As used herein, the term photostructure means that the surface bonded, continuous copolymer layer, which when spatially and temporally exposed to ultraviolet (UV) radiation/light of a sufficient intensity compared to the regions that are not exposed, leads to comparatively deep, well defined etched features called microstructures. The term "microstructure" as used herein is defined to have its conventional meaning, for example, the microscopic structure of a material including, phase boundaries, orientations, size scale and surface morphology including structures that have small features of dimensions measured in the micrometer range or patterned structures comprising "microscale" features. The terms "microscale", "micro-sized" or "micro-range" are to be interpreted broadly to include any dimensions that are in the range of about 1 µm to about 100 µm.

In other embodiments, the photostructuring of the surface-bonded copolymer layer is carried out by a selective UV exposure involving the use of a photomask. The term "photomask" is intended to mean any material having varying or non-uniform barrier properties to ultra-violet light or other electromagnetic radiation used to photopolymerize the copolymer layer of the invention. A preferred photomask material comprises an electromagnetic barrier material having a design which perforates (or is cut out of) the material. Upon application of electromagnetic radiation on one side of the photomask, a pattern of electromagnetic radiation is emitted from the opposite side of the photomask. The emitted pattern preferably comprises "shadow portions" (having virtually no electromagnetic radiation) and electromagnetic radiation portions: together the two portions can form an intricate pattern of electromagnetic radiation. The photomask used in the present invention can comprise openings of any size and shape known to a skilled person in the art.

The photomask used in the UV exposure according to any of the above described embodiments can be used to obtain a monolithic topography. The term "topography" as described herein refers to the relief features or surface configuration of an area or adjacent areas and encompasses depth which includes, but is not limited to, a geometrical depth and/or a depth of structure, or physical characteristic (physical attribute). Topography as used herein may include ridges, cones, pillars, hills, valleys, grooves, pits, dimples, depressions, bumps, convexity, concavity, ribs, protrusions, curves, raised surfaces or other surface topography. The terms "pillar" or "pillar-shaped" is to be interpreted broadly to include any substantially upright longitudinal body where the length is much greater than the width and where the width dimension is relatively constant throughout the length dimension. The terms "cone" or "cone-shaped" is to be interpreted broadly to include any substantially upright longitudinal body where the length is much greater than the width and where the width dimension decreases as the structure protrudes out of the surface where it is fixed. The terms "hills" or "hill-like structure" is to be interpreted to include a protrusion out of a surface where the width is much greater than the height and where the width dimension decreases as the structure protrudes out of the surface where it is fixed. In one embodiment, the UV exposure according to any of the above described embodiments provides for a monolithic topography, wherein the monolithic topography is preferably characterized by hill-like structures in a hexagonal arrangement. Such hill-like structures may comprise a height between 1 to 10 µm, preferably between 1 to 9 µm, preferably between 1 to 8 µm, preferably between 1 to 7 µm, preferably between 1 to 6 µm, preferably between 1 to 5 µm, preferably between 1 to 4 µm, preferably between 1 to 3 µm, preferably at least 1 µm and preferably 3 µm, even more preferably 2.5 µm.

In preferred embodiments, the photomask used in the UV exposure creates topography comprising circular openings separated by circular distances in a hexagonal arrangement. The photomask used in the UV exposure may comprise circular openings with a diameter and/or width between 10 to 50 µm, preferably 20 to 40 µm, even more preferably 25 to 35 µm, and most preferably 30 µm. Furthermore, the photomask used in the UV exposure comprises circular distances with a diameter between 10 to 50 µm, preferably between 20 to 40 µm, even more preferably between 20 to 30 µm, and most preferably 25 µm. Accordingly, in various embodiments, the photomask used in the UV exposure comprises circular openings separated by circular distances in a hexagonal arrangement, wherein the circular openings comprise a diameter of 30 µm, and wherein the circular openings are separated by circular distances with a diameter of 25 µm.

The present invention provides a continuous, surface-bonded, photostructured, copolymer layer according to the methods described herein. In preferred embodiments, the copolymer is an alternating copolymer. In other preferred embodiments, the copolymer is a one-component compound. In further preferred embodiments, the copolymer is a one-component compound, preferably wherein the copolymer is an alternating copolymer.

According to the third aspect of the invention, provided herein is a method of 3D functionalization of a container using the surface-bonded continuous layer of a copolymer of the invention. 3D functionalization of a container as used herein refers to selective spatial and temporal exposure of the copolymer layer using a photomask to form a 3D functionalized copolymer layer. Accordingly, in various embodiments, provided herein is a method of producing a copolymer layer with tailored 3D surface properties by functionalization. As described elsewhere herein, the method of manufacturing a container comprising a surface-bonded continuous copolymer layer includes selective UV exposure that allows the copolymer to form a cross-linked copolymer and further allows covalent bonding to the surface in order to produce a monolithic coating via the photo-reactive side group of a photo reactive crosslinker described herein for a C, H-insertion cross-linking reaction.

The present invention provides a surface-bonded continuous layer of a copolymer manufactured or obtainable or obtained by any of the methods and the corresponding embodiments disclosed herein.

The surface-bonded continuous layer of a copolymer manufactured by the methods disclosed herein shows a very high, reproducible binding capacity due to the click-binding reactive molecules disclosed herein. In various embodiments, the surface-bonded continuous layer of the invention provides a binding capacity of any of between 100 pmol to 140 pmol, between 110 pmol to 140 pmol, 120 pmol to 140 pmol, 130 pmol to 140 pmol, 135 pmol to 140 pmol, and even more than 140 pmol. These values provide for an increase in binding specificity by a factor of 5 to 7 times as compared to other copolymer layers known in the art. In a related embodiment, the binding capacity of the copolymer layer is directly dependent on the proportion of azide used. In various embodiments, azide at a concentration of 3.5 mol% gives the maximum binding capacity. The binding capacity is 0 pmol without any azide group.

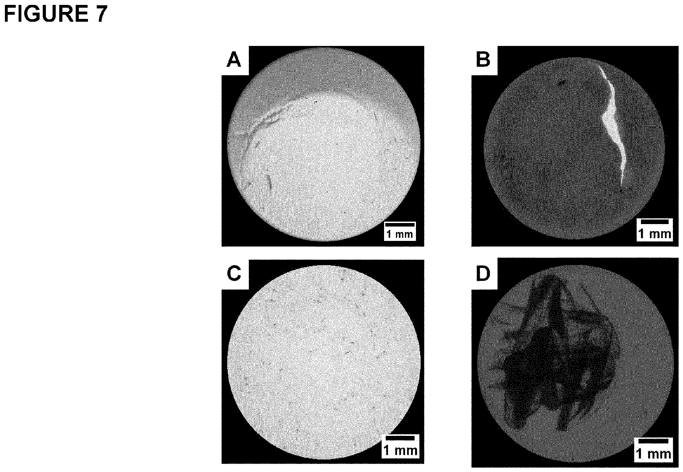

The surface-bonded continuous layer of a copolymer manufactured by the embodiments of the methods disclosed herein shows a significantly improved mechanical stability as shown in Example 4 and Figure 7.

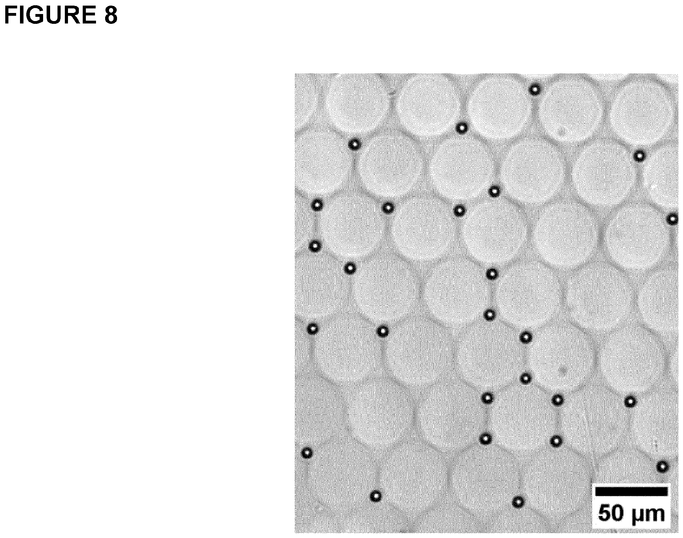

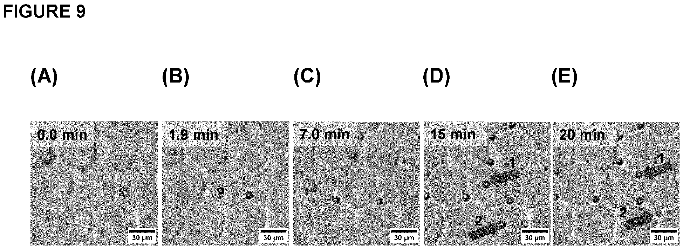

The surface-bonded continuous layer of a copolymer manufactured by the embodiments of the methods disclosed herein specifically with the 3D functionalization of photostructures explained herein are surprisingly sufficient to separate objects, particles and/ or cells. In various embodiments, these structures can separate particles with a diameter of 7 µm as shown in Figure 8 and Figure 9. In such embodiments, the cells can be separated over an interval of time wherein the separation is achieved between 0 to 20 minutes, 1.5 to 20 minutes, 7 to 20 minutes, preferably between 15 to 20 minutes. In such embodiments, the particles fall may largely into the micro-pockets created by the photostructured copolymer layer of the invention.

The surface-bonded continuous layer of a copolymer manufactured by the methods disclosed herein results in an improved sensitivity in detecting the analytes of interest. In various embodiments, the sensitivity is improved by a factor of 10 as compared to other copolymer layers known in the art. In such embodiments, the detection sensitivity of the copolymer layer of the invention is at least 0.5 µg / ml.

The invention provides a container having a surface-bonded continuous layer of a copolymer manufactured or obtainable or obtained by the methods disclosed herein. Preferably, the container has the continuous layer of a copolymer bonded to the surface of the bottom end of the container. In various embodiments, the container is formed of polymeric material and/or may have an open top end, a closed bottom end, and at least one wall extending between said ends. The surface of the bottom end may have a surface-bonded continuous layer of a copolymer comprising at least three different monomer units and including
(i) at least one photo-reactive side group for a C, H-insertion cross-linking ["CHic"] reaction; and
(ii) at least one click binding-reactive side group ["Click"] for (bio-)functionalizing of the copolymer layer.

The copolymer layer of the container may be photostructured while maintaining a monolithic topography, preferably the copolymer layer has hill-like structures. Preferably, the hill-like structures exhibit a hexagonal arrangement.

The container having a surface-bonded continuous layer of a copolymer manufactured by the embodiments of the methods disclosed herein shows a very high, reproducible binding capacity due to the click-binding reactive molecules disclosed herein. In various embodiments, the surface-bonded continuous layer of the invention provides a binding capacity between 100 pmol to 140 pmol, between 110 pmol to 140 pmol, 120 pmol to 140 pmol, 130 pmol to 140 pmol, 135 pmol to 140 pmol, or even more than 140 pmol. These values provide for an increase in binding specificity by a factor of 5 to 7 times as compared to other copolymer layers known in the art. In further embodiments, the binding capacity of the copolymer layer is directly dependent on the proportion of azide used. In various embodiments, azide at a concentration of 3.5 mol% gives the maximum binding capacity. The binding capacity is 0 pmol without any azide group.

The container having a surface-bonded continuous layer of a copolymer manufactured by the embodiments of the methods disclosed herein shows a significantly improved mechanical stability as shown in Example 4 and Figure 7.

The container having a surface-bonded continuous layer of a copolymer manufactured by the embodiments of the methods disclosed herein specifically with the 3D functionalization of photostructures explained herein are surprisingly sufficient to separate objects, particles and/ or cells. In various embodiments, these structures can separate particles with a diameter of 7 µm as shown in Figure 8 and Figure 9. In such embodiments, the cells can be separated over an interval of time wherein the separation is achieved between 0 to 20 minutes, 1.5 to 20 minutes, 7 to 20 minutes, preferably between 15 to 20 minutes. In such embodiments, the particles fall largely into the micro-pockets created by the photostructured copolymer layer of the invention.

The container having a surface-bonded continuous layer of a copolymer manufactured by the embodiments of the methods disclosed herein results in reduced non-specific background reactions and an improved sensitivity in detecting the analytes of interest. In various embodiments, the sensitivity is improved by a factor of 10 as compared to other copolymer layers known in the art. In such embodiments, the detection sensitivity of the copolymer layer of the invention is at least 0.5 µg / ml.

In the present invention, the container may comprise an optically transparent part. In various embodiments, the container has an essentially tubular shape and/or has a conical shape towards the bottom end of the container, preferably the container is a laboratory test tube, e.g. a well of a microplate.

The present invention provides a container assembly, or an analytical device, comprising one or more of a surface-bonded continuous layer of a copolymer as disclosed herein, or one or more of a container as disclosed herein.

The present invention provides the use of the surface-bonded continuous layer of a copolymer according to the invention, or the container of the invention, or the container assembly or analytical device of the invention, in analytical and/or diagnostic applications, preferably for use in an immunoaffinity assay, ELISA or ELISpot assay. In various embodiments, the said use is for separation of dispersed particles and/or objects, preferably wherein the dispersed particles and/or objects are cells.

The present invention provides a method of using the surface-bonded continuous layer of a copolymer according to the invention, or the container of the invention, or the container assembly or analytical device of the invention, in analytical and/or diagnostic applications, preferably for use in an immunoaffinity assay, ELISA or ELISpot assay.

The term "immunoaffinity" means techniques by which materials are isolated by virtue of their content of immunogenic epitopes. In a well-known immunoaffinity assay, ELISA, a capture antibody is printed on the bottom of a well in a microtiter plate. A microtiter plate is a flat plate that has multiple containers or "wells" formed in its surface. Each well can be used as a small test tube into which various materials can be placed to perform biochemical analyses. In a preferred embodiment, the bottom of the container, wells and/or test tube is optically transparent. By the term "optically transparent" as used herein is meant an object having a transmittance of at least 50% at a wavelength in the visible spectrum (380 nM-780 nM). The capture antibody used in ELISA has specificity for a particular antigen for which the assay is being performed. A sample to be analyzed is added to the well containing the capture antibody, and the capture antibody "captures" or immobilizes the antigen contained in the sample. A detect antibody is then added to the well, which also binds and/or forms a complex with the antigen. Further materials are then added to the well which cause a detectable signal to be produced by the detect antibody. For example, when light of a specific wavelength is shone upon the well, the antigen/antibody complexes will fluoresce. The amount of antigen in the sample can be inferred based on the magnitude of the fluorescence. In another example, a compound can be added to the well that causes the detect antibody to emit light within a predetermined wavelength (e.g., 400-500 nm). This light can be read by a charge-coupled device (CCD) camera to measure the optical brightness of the emitted light. Example 6 demonstrates the use of the surface-bonded continuous layer of a copolymer forming part of the method of the invention in an ELISA plate by measuring IL-6 as an analyte of interest. The ELISpot (Enzyme-linked-immuno-Spot) test is a sensitive method to measure cell activation at the single cell level. This cell activation releases cytokines, which are then made visible as colored dots (spots) in the ELISpot using monoclonal antibodies. With regard to the detection of cytokines that were secreted by cells, the ELISPOT method appears to be more sensitive by up to a factor of 200 than a comparable ELISA method. A well-known problem with analytical techniques such as ELISPOT technology is the creation of clearly defined spots after the detection reaction has been completed. Spot generation is influenced, among other things, by high non-specific background signals, a "washing out" of the edges and the mixing of the individual spots into clusters. Here, however, cells that are close together can also cause interference of the spots that form. The present invention has surprisingly overcome this problem by providing a container having a surface-bonded, continuous layer of copolymer that is capable of separation of dispersed particles and/or objects, preferably wherein the dispersed particles and/or objects are cells. The term "cell" as used herein may be any cell type. In certain embodiments, the cells are mammalian cells.

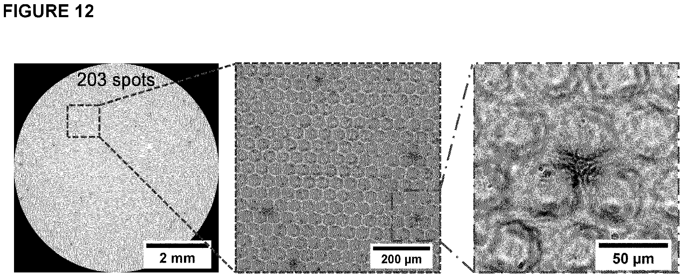

The use of the surface-bonded continuous layer of a copolymer manufactured by the method of the invention surprisingly provides for reduced non-specific background reactions as illustrated in Examples 5 and 6. As illustrated in Example 5, the surface-bonded continuous layer of a copolymer manufactured by the method of the invention provides for about twice as many spots as carried out without the copolymer of the invention in a reference plate. In addition, the detection signals, i.e., the color spots, are spatially clearly separated, sharper and smaller than on the reference plate (Figure 12).

As illustrated in Example 7, the surface-bonded continuous layer of a copolymer manufactured by the method of the invention provides for covalent attachment and/or capturing of biochemical reagents according to the analytical test used. The term biochemical reagent composition as used herein refers to a composition as a reagent for the detection, analysis or use as a substrate or intermediate in synthetic, biochemical, or immunological reactions e.g. emission reagents include luminescent, fluorescent, or phosphorescent materials. Other emission reagents include colloidal gold, colloidal silver, other colloidal metals and non-metals, quantum dots, other fluorescent nanoparticles, plasmon resonant particles, grating particles, photonic crystals reagents and the like. Furthermore, absorption reagent refers to any material that causes absorbance of the excitation light. Example 7 illustrates the covalent attachment of streptavidin-dibenzocyclooctin. In this way, the methods of the invention provide for testing the binding specificity of any biochemical reagent on the copolymer manufactured by the method of the invention.

The means and methods of the present invention provide numerous advantages including, but not limited to:
The binding capacity clearly exceeds that of other 3D coatings, which are usually only produced by individual branched polymer chains. The invention provides for a high, reproducible, specific binding capacity for any, but specifically selected molecules.

Due to the increased specific binding capacity, small, spatially clearly recognizable spots could be achieved in the ELISpot. This also increased sensitivity.

The modified copolymer surface by the compositions of the invention is characterized by increased chemical and mechanical stability compared to the otherwise usual filter base plates.

The copolymer forming part of the invention significantly reduces non-specific background reactions. Fewer washing and blocking steps are necessary or can be carried out with milder reagents.

Using photomasks and a unique UV exposure, the polymer can be specifically shaped into a microstructured hydrogel. This also made it possible to separate and selectively distribute objects or cells.

### EXAMPLES

The invention is illustrated in the following examples. These examples are provided for illustrative purposes only and are not to be considered to limit the scope of the invention to any extent.

### Example 1 - Preparation of compounds for modifying a surface

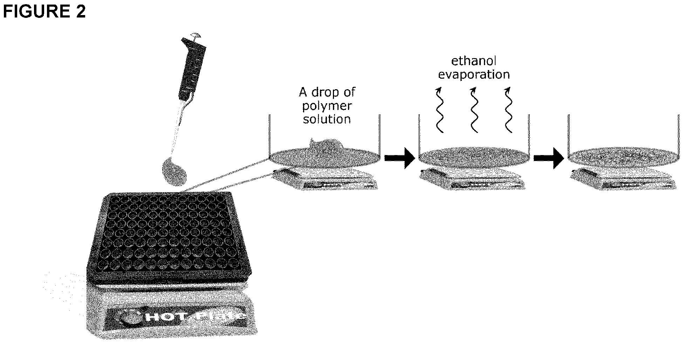

Figure 1 shows an example of a structural formula of the synthetic one-component compound (copolymer) with which the plastic surface can be modified accordingly. The letters x, y and z indicate the variable proportions of the monomer units. An advantageous composition would be, for example, x = 94 mol%, y = 2.5 mol% and z = 3.5 mol%. The number average molar mass is, for example, 25,000 g / mol. The one-component compound is dissolved in pure ethanol, whereby a concentration of 100 mg / ml is obtained. Then 2 µl of this solution are pipetted onto the bottom of a container, here the bottom of a well of a microtiter plate made of polystyrene, which was heated to 70 ° C. The volume of the solution spreads out on the surface of the bottom of the well, with the solvent evaporating rapidly at the same time. When the edge of the well is reached, the solvent has evaporated, a uniform layer having formed on the surface. Figure 2 shows a schematic representation of a coating of microtiter plates using a manual dispensing process. Further application examples for the production of the modified surface with ethanol as solvent, a concentration of 100 mg / ml and a volume of 2 µl are summarized in Table 1.

**Table 1: List of coating parameters (copolymer composition, number-average molar mass M n and coating temperature) for the production of modified surfaces.**

| **polymer** | **Mₙ [g / mol]** | **coating temperature [° C]** |
|---|---|---|
| PDMAA 2.5% MABP | ≈ 43,000 | ≈ 53 |
| PDMAA 2.5% MABP-3.5% AZMA | ≈ 25,000 | ≈ 70 |
| PDMAA 2.5% MABP 5.5% AZMA | ≈ 65,000 | ≈ 35 |
| PDMAA 2.5% MABP 7.5% AZMA | ≈ 39,000 | ≈ 45 |
| PDMAA 2.5% MABP-8.5% AZMA | ≈ 49,000 | ≈ 47 |

### Example 2 - UV Exposure - crosslinking and covalent bonding to the surface

The coatings obtained from the method of Example 1 are exposed by UV exposure at a wavelength of, for example, 365 nm with an energy of, for example, 4 J / cm 2 , as a result of which the one-component compound is crosslinked and covalently bonded to the plastic surface, so that a monolithic coating is produced.

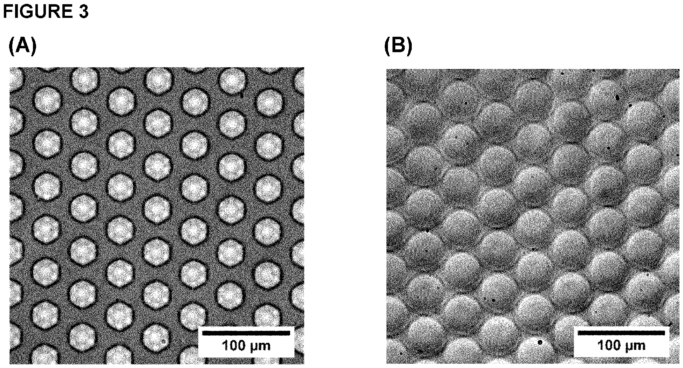

If a photomask is used in the UV exposure, which has hexagonal, circular openings with a diameter of, for example, 30 µm, the openings being separated by circular distances with a diameter of 25 µm, a monolithic topography with a columnar structure on the plastic surface can be obtained which is shown in Figure 3.

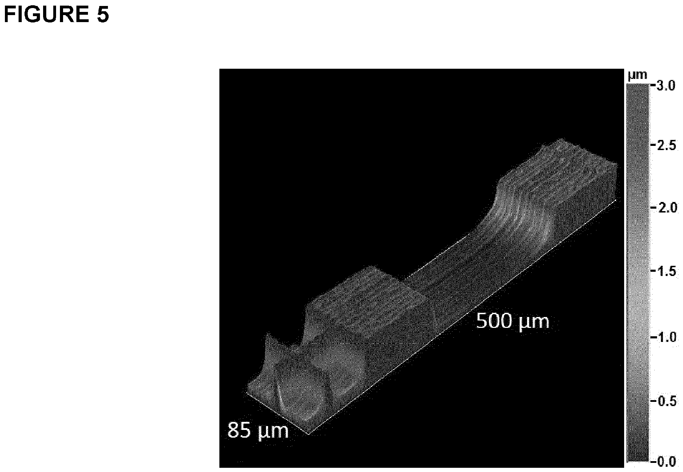

In order to demonstrate the formation of a monolithic coating by this process, the topography was examined using an atomic force microscope [atomic force microscopy (AFM)]. Figure 4 shows an example of the result of such a measurement. A total layer thickness of 2.5 µm can be seen from the height profile shown. Furthermore, a continuous coating is achieved, since there is also cross-linked copolymer between the columns. This was an unexpected but desirable result because it created a complete, coherent layer. This could be confirmed by measurements on the mechanical profilometer (Figure 5).

### Example 3 - Binding Capacity Measurement

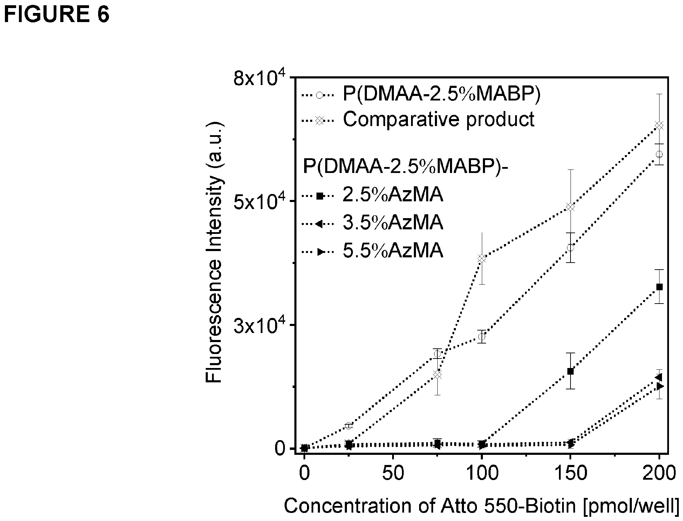

Binding capacity measurements of these surfaces showed a very high, reproducible binding of click-binding-reactive molecules compared to other 3D coatings, which are usually generated by single branched polymer chains (Figure 6). Binding capacities of more than 140 pmol / well were achieved, with azide contents of approximately 3.5 mol% in the copolymer. A comparable coating of a product on the market has a binding capacity of about 20 pmol / well. An increase by a factor of 7 could thus be achieved. A straight line of origin results for a copolymer without clickable groups, i.e. the binding capacity is, as expected, 0 pmol / well.

### Example 4 - Mechanical Stability

The modified plastic surface shows a significantly improved mechanical stability compared to the Filter floor panels for ELISpot tests. This was tested by specifically scratching with a pipette (tip diameter about 1 mm) on the plate bottoms (Figure 7). The modified plastic surface withstands high pressure (about 14 g/mm², Figure 7 A), whereby the plate bottom of the filter base plate breaks through (Figure 7 B). With light pressure (about 7 g/mm²) with the pipette on the plate bottom, the monolithic coating only leads to a reduction in the signals (Figure 7C), whereas unwanted flat signals are generated with the reference plate (Figure 7 D).

### Example 5 - Cell Separation

The structures obtained in this way can be used very well to separate, for example, polystyrene particles with a diameter of 7 µm on the modified plastic surface (Figure 8). The sedimentation process of Peripheral Blood Mononuclear Cells (PBMC) is shown in Figure 9. It can be seen that PMBCs that hit a column migrate into a micro-pocket in a relatively short time and can thus be separated. The unique and unexpected thing about it is that the particles/cells could be separated purely by the topography. The entire surface consists only of the one-component compound and both the columns and the micro-pockets have the same chemical structure.

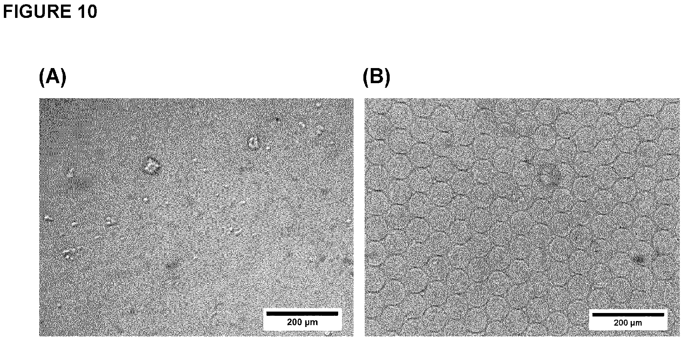

Cell separation during the ELISpot test is illustrated in Figure 10. Cell clusters can be seen on the unstructured one-component copolymer. In the case of the microtiter plate base coated and structured with the one-component copolymer, the cells fall largely into the micro-pockets intended for this purpose.

### Example 6 - Sensitivity Measurement in ELISpot

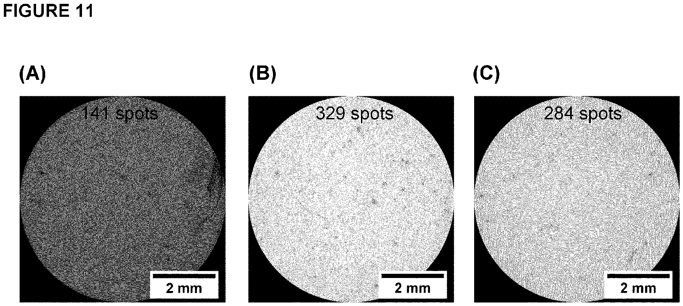

Coating with the one-component compound results in a (diagnostic) sensitivity of the ELISpot test that is improved by a factor of about 10. This was shown by a titration series of PHA-M (stimulates cell proliferation), whereby, compared to the reference plate (5.0 µg / ml), 0.5 µg / ml was still detectable with the coating (Table 2). Figure 11 shows, for example, a test series with 30 µg / ml PHA-M, in which it can be seen that with the ELISpot assay carried out in the same way, about twice as many spots are obtained. The comparatively lower non-specific background coloration in the coated well is also clear. In addition, the detection signals, i.e. the color spots, are spatially clearly separated, sharper and smaller than on the reference plate. The usual spot diameter on the reference plate is 75 - 400 µm, which corresponds to other standard ELISpot plates. Spots with a diameter of around 30 µm can be clearly assigned to exactly one cell on the structured one-component copolymer (Figure 12).

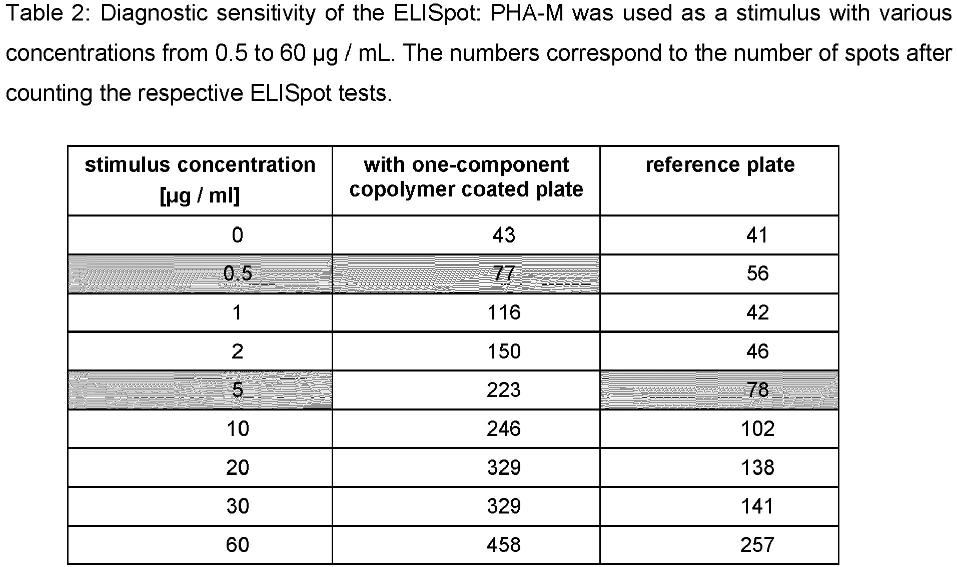

### Example 6 - IL-6 measurement in ELISA

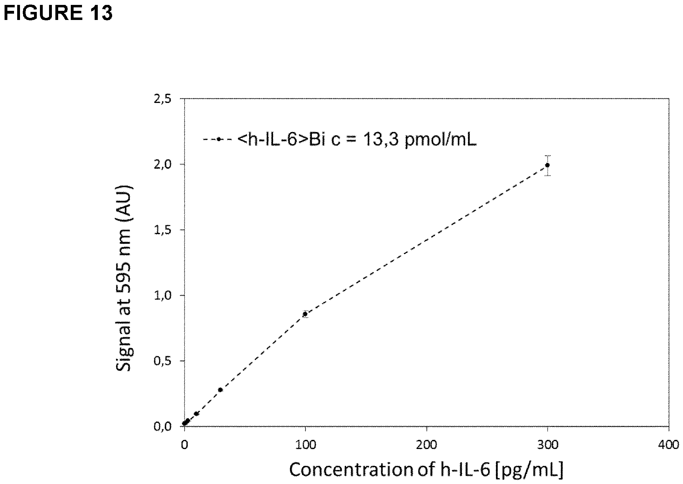

The surface bonded continuous copolymer layer used in this example is similar to Examples 1 and 2 with minor modifications. The copolymer is also dissolved in ethanol and with a dispenser (e.g., Multidrop from Thermo Fisher Scientific) in a 100 mg / mL solution that has been heated to 45°C, with 2 µL dispensed evenly per well on microtiter plates (MTP) from Greiner Bio One (655906). After the ethanol has evaporated, the MTP are irradiated with UV light (UV table TFX-20M from Vilber-Lourmat) until after one minute the polymer has cross-linked. With repeated washing with ethanol, unbound polymer strands are removed. The azide hydrogel MTP thus obtained is coated in a second step with streptavidin-dibenzocyclooctyne (SA-DBCO) from Microcoat Biotechnologie GmbH. A biotinylated antibody against IL-6 with 0.2 µg / well is bound to the corresponding SA plate (also Microcoat Biotechnologie GmbH). Solutions with different concentrations of human IL-6 are placed on this plate as samples. This IL-6 is in turn bound with an antibody against IL-6 which has been labeled with peroxidase. A color reaction is initiated by means of TMB, by means of which the wells are stained in accordance with the amount of bound IL-6. This color reaction is stopped with dilute sulfuric acid and measured in an MTP reader (e.g., from Biorad) at 450 to 595 nm. The IL-6 ELISA works very well (see Figure 13) with an LOD of 0.81 pg / mL and a LOQ of 1.64 pg / mL. This also shows the very low background of the hydrogel plates, which is comparable to a pure buffer measurement in an MTP.

### Example 7 - Binding capacity of streptavidin

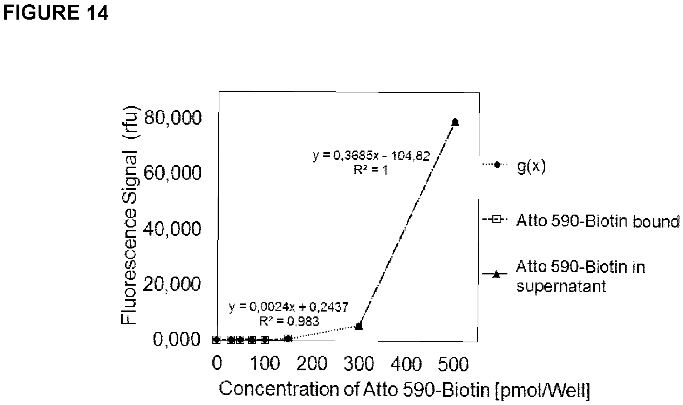

The hydrogel plate-based SA-MTP from Example 2 were also used for a binding capacity test. The aim is to demonstrate how many binding sites for biotin exist on the plate and thus to draw conclusions about the bound amount of streptavidin. For this test, Atto 590-Biotin (Sigma #43208) dissolved in DMSO was first applied to the SA-MTPs with different amounts per well between 0 and 500 pmol. The Atto 590-Biotin binds completely to the plate below the binding capacity of the plate (examined plate). The excess no longer binds to the plate above the binding capacity. After an incubation period of 2 hours with shaking at room temperature and exclusion of light, part of the supernatant was transferred from this plate to another plate (measuring plate). This supernatant was then measured with a fluorescence reader (Fluoroscan Ascent) at an excitation wavelength of 584 nm and a detection at 620 nm. The results of this are shown in Figure 14. As expected, no signals can be seen in the binding capacity test at lower concentrations. At the higher concentrations, the signal in the measuring plate rises because no Atto 590-Biotin has bound to the plate under investigation. A trend line can be formed from all points at which Atto 590-Biotin is bound to the plate, and another trend line from the points at which Atto 590-Biotin is detected in the supernatant. The concentration of Atto 590-Biotin at which the two straight lines intersect is the binding capacity of the plate. In the selected case this is 287 pmol / well. This means that up to 287 pmol biotin can be bound in one well of the hydrogel plates. Assuming that up to four binding sites of SA immobilized are still capable of binding, it can be calculated that at least 71 pmol SA were immobilized in the plate.

## Claims

1. A method of manufacturing a surface-bonded continuous layer of a copolymer, comprising the steps:
(a) providing a copolymer dissolved in a solvent, wherein the copolymer comprises at least three different monomer units and includes
(i) at least one photo-reactive side group for a C, H-insertion cross-linking ["CHic"] reaction; and
(ii) at least one click binding-reactive side group ["Click"] for (bio-) functionalizing of the copolymer layer;
(b) applying the dissolved copolymer of (a) to a surface and forming a continuous copolymer layer on the said surface;
(c) after evaporation of the solvent, exposing the continuous copolymer layer of (b) to UV light for cross-linking [of the copolymer (chains)] and binding [of the cross-linked copolymer] to the said surface.

2. A method of manufacturing a container having a continuous layer of a copolymer bonded to the surface of the bottom end of the container, comprising the steps:
(a) providing a copolymer solved in a solvent, wherein the copolymer comprises at least three different monomer units and includes
(i) at least one photo-reactive side group for a C, H-insertion cross-linking ["CHic"] reaction; and
(ii) at least one click binding-reactive side group ["Click"] for (bio-) functionalizing of the copolymer layer;
(b) applying the dissolved copolymer of (a) to the surface of the bottom end of a container and forming a continuous copolymer layer on the said surface;
(c) after evaporation of the solvent, exposing the continuous copolymer layer of (b) to UV light for cross-linking and binding to the said surface.

3. The method of claim 2, wherein the container is formed of polymeric material and has an open top end, a closed bottom end, and at least one wall extending between said ends.

4. The method of any one of claims 1 to 3, wherein the amount of copolymer applied to the surface is at least 0.5 µg/mm², preferably at least 8-10 µg/mm².

5. The method of any one of claims 1 to 4, further comprising a step of photostructuring the surface-bonded copolymer layer by a spatially selective exposure of the copolymer layer to UV light.

6. The method of claim 5, wherein the photostructuring generates hill-like structures on the continuous copolymer layer while maintaining a monolithic topography, preferably wherein the hill-like structures exhibit a hexagonal arrangement.

7. The method of claim 5 or 6, wherein the hill-like structures have a diameter of 10-50 µm and/or a height of 1-10 µm.

8. A surface-bonded continuous layer of a copolymer obtainable by the method of any one of claims 1 and 4 to 7.

9. A container having a continuous layer of a copolymer bonded to the surface of the bottom end of the container obtainable by the method of any one of claims 2 to 7.

10. A container formed of polymeric material and having an open top end, a closed bottom end, and at least one wall extending between said ends, **characterized in that** the surface of the bottom end has a surface-bonded continuous layer of a copolymer comprising at least three different monomer units and including
(i) at least one photo-reactive side group for a C, H-insertion cross-linking ["CHic"] reaction; and
(ii) at least one click binding-reactive side group ["Click"] for (bio-)functionalizing of the copolymer layer.

11. The container of claim 10, wherein the copolymer layer is photostructured while maintaining a monolithic topography, preferably wherein the copolymer layer has hill-like structures, preferably wherein the hill-like structures exhibit a hexagonal arrangement.

12. The method of any one of claims 2 to 7, or the container of any one of claims 9 to 11, wherein the container comprises an optically transparent part, and/or wherein the container has an essentially tubular shape and/or has a conical shape towards the bottom end of the container, preferably wherein the container is a laboratory test tube, e.g. a well of a microplate.

13. The method of any one of claims 1 to 7, or the surface-bonded continuous layer of a copolymer of claim 8, or the container of any one of claims 9 to 12, wherein the at least three different monomer units comprise a hydrophilic monomer, preferably a hydrophilic acryl monomer, and/or wherein at least one monomer is a photoreactive crosslinker, and/or wherein the at least one click binding-reactive side group is an azide or a derivative thereof.

14. A container assembly, or an analytical device, comprising one or more of a surface-bonded continuous layer of a copolymer according to claim 8, or one or more of a container according to any one of claims 9 to 13.

15. Use of the surface-bonded continuous layer of a copolymer according to claim 8, or the container of any one of claims 9 to 13, or the container assembly or analytical device of claim 14, in analytical and/or diagnostic applications, preferably for use in an immunoaffinity assay, ELISA or ELISpot assay.

16. The use of claim 15, which is for
(a) separation of dispersed particles and/or objects, preferably wherein the dispersed particles and/or objects are cells; or
(b) the selective covalent attachment and/or capturing of biochemical reagents to the copolymer layer.
